(19) Europäisches Patentamt
European Patent Office
Office européen des brevets

(11) Publication number: 0 108 452 A1

# (12) EUROPEAN PATENT APPLICATION

(21) Application number: 83201551.5

(22) Date of filing: 28.10.83

(51) Int. Cl.[3]: A 61 K 31/557
A 61 K 45/06
//(A61K31/557, 31/415)

(30) Priority: 05.11.82 US 439472

(43) Date of publication of application: 16.05.84 Bulletin 84/20

(84) Designated Contracting States: BE CH DE FR IT LI LU NL SE

(71) Applicant: THE PROCTER & GAMBLE COMPANY
301 East Sixth Street
Cincinnati Ohio 45201(US)

(72) Inventor: Wagner, Gregory Steven
5847 Sigmon Way
Fairfield, OH 45014(US)

(72) Inventor: Matthews, Randall Stryker
1437 Forester Drive
Cincinnati, OH 45240(US)

(74) Representative: Suslic, Lydia et al,
Procter & Gamble European Technical Center Temselaan 100
B-1820 Strombeek-Bever(BE)

## (54) Treatment of gastric inflammatory disease with cytoprotective prostaglandins and histamine-2 blocking anti-secretory agents.

(57) Compositions comprising gastric cytoprotective prostaglandin or prostaglandin-like compounds and histamine-2 receptor blocking anti-secretory agents useful in the treatment and prophylaxis of gastric inflammatory conditions are disclosed. These compositions are effective in the treatment and prophylaxis of gastro-intestinal ulceration. They utilize levels of both prostaglandin and anti-secretory agents which are significantly lower than ordinarily required as the prostaglandin potentiates the effect of the anti-secretory agent, and minimizes the side effects which are frequently associated with the administration of prostaglandins. The method of treating and preventing gastric inflammatory diseases using these compositions is also disclosed.

EP 0 108 452 A1

# TREATMENT OF GASTRIC INFLAMMATORY DISEASE WITH CYTOPROTECTIVE PROSTAGLANDINS AND HISTAMINE-2 BLOCKING ANTI-SECRETORY AGENTS

Gregory S. Wagner
Randall S. Matthews

## TECHNICAL FIELD

This invention relates to compositions and methods for the treatment and prophylaxis of certain gastric inflammatory diseases comprising the utilization of certain prostaglandin and prostaglandin-like compounds in combination with certain histamine-2 receptor blocking type anti-secretory agents. This combination of agents is useful in the treatment and prophylaxis of certain inflammatory diseases of the stomach and duodenum. Further, the invention permits the administration of certain prostaglandin and prostaglandin-like gastric cytoprotective agents in combination with histamine-2 receptor blocking anti-secretory agents at levels below which either agent would ordinarily demonstrate clinical efficacy against these diseases with a corresponding reduction in side effects.

## BACKGROUND OF THE INVENTION

Prostaglandins and prostaglandin analogues are well-known in the treatment of gastro-intestinal tract disorders. U.S. Patent 3,903,297, Robert, issued September 2, 1975, and U.S. Patent 3,781,429, Partridge, et al., issued December 25, 1973, describe the use of prostaglandin-like compounds to reduce gastric secretion, and accordingly treat ulceration of the gastro-intestinal tract. The use of prostaglandin-like compounds in combination with agents which are known to cause gastric damage or disease when administered without prostaglandins is also known. For example, the concommitant use of prostaglandin-like compounds with non-steroidal anti-inflammatory compounds is known to be effective in reducing the undesirable gastro-intestinal side effects

which ordinarily accompany the administration of the non-steroidal anti-inflammatory agents. See U.S. Patents 3,911,124, Robert, issued October 7, 1975; 3,917,828, Robert, issued September 4, 1975; 3,928,588, Robert, issued December 23, 1975; and 3,927,213, Lippmann, issued December 16, 1975. M.M. Cohen has described the use of a prostaglandin ($PGE_2$) in preventing damage to the gastric mucosal barrier caused by non-steroidal anti-inflammatory compounds such as aspirin and indomethacin in the dog. Gastroenterology 68:876 (1975).

The treatment and prevention of gastric damage and gastric erosive diseases, such as ulceration, with other therapies is also well-known. For example, Mann, Gastroenterology 68; 946 (1976) describes the prevention of acute erosive gastritis by antacid administration.

The treatment of gastric inflammatory disease with agents other than prostaglandins is likewise well-known in the art. Cimetidine, (successfully marketed under the tradename Tagamet), is an anti-secretory agent widely used in the treatment of gastric ulcers. This compound is thought to act by blocking the histamine receptors within the stomach mucosa (labelled histamine-2 or H-2 receptors, to distinguish from those histamine receptors generally associated with allergic response) thereby preventing histamine molecules from signaling stomach cells to secrete acid. Other histamine-2 antagonist drugs, (i.e., those which block the H-2 receptors) such as ranitidine, which are either more potent and/or longer acting than cimetidine, are also well-known. See C&E News, April 12, 1982, pp 24-26.

The use of prostaglandin agents, capable of inhibiting acid secretion, at non-anti-secretory doses, is also well-known. See U.S. Patent 4,097,603, Robert, issued June 27, 1978.

There exists among the many prostaglandin compounds, and analogues thereof, a subclass of prostaglandin compounds which exhibits gastric cytoprotection. Within this subclass of gastro-cytoprotective prostaglandins is a smaller subclass which is additionally anti-secretory, in addition to possibly being cytoprotective. There also exists, however, a heretofore

unsolved problem, with both the gastric cytoprotective as well as the anti-secretory-cytoprotective prostaglandins, which has effectively prevented true therapeutic utility; there are numerous side effects (such as diarrhea) which are associated with the use of prostaglandins administered at therapeutically useful levels. And, as is the case with almost all therapeutic agents, both the number and severity of the side effects of the particular agent or agents increase as the level and frequency of administration increase.

While there are prostaglandin-like compounds which demonstrate both gastric cytoprotective properties as well as anti-secretory properties, the level and frequency of administration required to achieve cytoprotection is generally significantly lower than the level of administration needed to achieve significant reductions in acid secretion. See U.S. Patent 4,097,603, Robert, issued June 27, 1978. Accordingly, when the same compound exhibits both gastric cytoprotective properties and anti-secretory properties, the administration of that compound at an anti-secretory level will result in an increase in both number and severity of side effects when directly contrasted with a level which simply shows gastric cytoprotection.

Further, while the level of administration needed to demonstrate a significant level of gastric cytoprotection can be shockingly small, (See Robert, A., et al.,; Cytoprotection by Prostaglandins in Rats; Prevention of Gastric Necrosis Produced by Alcohol, HCl, NaOH, Hypertonic NaCl and Thermal Injury. Gastroenterology 77: 43 (1979), where it is demonstrated that a fraction of a microgram can be effective), even administration at these low levels can frequently cause such side effects as stimulation of the uterus, nausea, retching, vomiting, stimulation of intestinal motility, abdominal discomfort, diarrhea, and fever. See Karim, et al.,: Effects of Some Natually Occurring Prostaglandins and Synthetic Analogues on Gastric Secretion and Ulcer Healing in Man, Advances in Prostaglandin and Thromboxane Research 2; 529 (1976); Konturek, Prostaglandins and Gastrointestinal Secretion and Motility, Anal. Exp. Med. Biol. 106: 297 (1977); Madsen, Prostaglandins and Chronic Diarrhea:

Clinical Aspects, Scand. J. Gastroenterology 14, Suppl. 53: 73 (1979). Accordingly, a method of administering prostaglandins and prostaglandin-like compounds at levels below those which are presently recognized as being minimally required to demonstrate significant gastric cytoprotection is needed to make these compounds truly useful in a therapeutic setting. The effective administration at very low levels would, for the first time, allow a whole range of prostaglandin and prostaglandin-like compounds to be clinically useful for the treatment of peptic ulcers and other gastric inflammatory disease.

The general premise discussed above is equally applicable to histamine-2 blocking anti-secretory agents. While, unlike prostaglandins, cimetidine is presently being used by millions of people worldwide in the clinical treatment of gastric ulcers, the prolonged use of cimetidine is associated with many side effects which can detract from its significant ulcer healing and symptom relief. For example, it is known that cimetidine interacts with some commonly used drugs which are metabolized in the liver by oxidation. This effect causes a prolongation of their effect. Benzodiazepine has been reported to interact with cimetidine resulting in an increase in plasma $T_{\frac{1}{2}}$ and decreased elimination. In rats, fetal and neonatal cimetidine exposure reduces adult testicular and prostatic weight, lowers adult testosterone levels and disturbs sexual behavior. In clinical studies, cimetidine has been demonstrated to be a weak anti-androgen. Accordingly, cimetidine should not be prescribed to pregnant women, especially at high dosage levels. Further, single intravenous doses of cimetidine have been demonstrated to penetrate into the cerebrospinal fluid. In the volume Worldwide Tagamet Experience published by Smith, Kline and French International Company, distributed at the World Congress of Gastroenterology, June, 1982, Stockholm, Sweden, the adverse reactions to cimetidine therapy which were reported (with varying frequency) included gynecomastia, decrease in white blood cell count, aplastic anemia, reversible mental confusion, interstitial nephritis, and impotence and decreased libido among male receipients. The drug

interactions, in addition to those listed above, are the potentiation of anti-coagulents, as well as other interactions with propanolol, theophylline, and anticonvulsants.

In summary, while it is clear that the cimetidine is of great utility in the treatment of certain gastric pathologies, and the side effects of cimetidine are not as significant as those of prostaglandins and prostaglandin-like compounds, it is still clear that there are significant clinical risks associated with its administration. It is, therefore, only logical that a method which would allow the administration of cimetidine at levels below the levels presently required to demonstrate significant clinical effects in the treatment and prevention of ulcers is as highly desirable as a method which would allow the administration of prostaglandins at the lowest possible level.

It has now been found that the administration of certain cytoprotective prostaglandins and prostaglandin-like compounds, at non-anti-secretory dosage levels, and preferably at dosage levels which are too small to demonstrate maximal intrinsic activity in ordinary therapeutic regimens, in combination with the administration of histamine-2 receptor blocking anti-secretory agents, especially cimetidine, at below art-established maximal intrinsic activity levels, results in the potentiation of both agents allowing effective treatment of gastric inflammatory disease with significant reduction of the side effects of both agents.

It is, therefore, an object of the present invention to provide an effective method of treatment and prophylaxis of gastric inflammatory conditions by the oral administration of a prostaglandin/histamine-2 receptor blocking anti-secretory combination.

It is a further object of the present invention to provide an effective method of treatment and prophylaxis of inflammatory diseases of the stomach and the duodenum with a prostaglandin/histamine-2 receptor blocking anti-secretory composition where the agents are administered at levels significantly below those at which either agent demonstrates or provides maximal intrinsic activity in the treatment and prophylaxis of said disease.

It is still a further object of the present invention to provide a method for reducing the side effects which ordinarily accompany prostaglandin administration, and histamine-2 receptor blocking anti-secretory agent administration by potentiating their effect, allowing reduced therapeutic levels. This is accomplished by concurrently administering a prostaglandin or prostaglandin-like compound and a histamine-2 receptor blocking anti-secretory agent.

It is still a further object of the present invention to provide novel pharmaceutical compositions comprising a safe and potentiating amount of a prostaglandin or prostaglandin-like compound and a safe and potentiable amount of a histamine-2 receptor blocking anti-secretory agent useful in the treatment and prophylaxis of gastric inflammatory disease.

It is still a further object of the present invention to provide effective pharmaceutical compositions comprising a safe and potentiating amount of a prostaglandin or prostaglandin-like compound, and a safe and potentiable amount of a histamine-2 receptor blocking anti-secretory agent which allow both of these agents to be administered at levels significantly below those at which either agent provides or demonstrates maximal intrinsic activity in the treatment and prophylaxis of inflammatory diseases of the stomach and duodenum.

## SUMMARY OF THE INVENTION

The present invention provides novel pharmaceutical compositions and methods employing prostaglandins and prostaglandin-like compounds in the treatment and prophylaxis of gastric disease and pathology, principally gastritis and gastric, peptic or duodenal ulcers, by the utilization of gastric cytoprotective prostaglandin or prostaglandin-like compounds in combination with certain histamine-2 receptor blocking anti-secretory agents.

In particular, the invention provides compositions useful in

reducing or preventing gastric inflammatory disease in humans or lower animals, comprising

(a) a safe and potentiating non-anti-secretory amount of a gastric cytoprotective prostaglandin or prostaglandin-like compound; and

(b) a safe and potentiable amount of a histamine-2 receptor blocking anti-secretory agent;

wherein said gastric cytoprotective prostaglandin is present at a level below which it exhibits maximal intrinsic activity as a cytoprotective agent; and further wherein said histamine-2 receptor blocking anti-secretory agent is present at a level below which it exhibits maximal intrinsic activity as an anti-secretory agent.

Further, the invention provides methods for reducing and preventing gastric inflammatory disease in humans or lower animals which comprise administering to a human or lower animal who is at risk to said disease

(a) a safe and potentiating non-anti-secretory amount of a gastric cytoprotective prostaglandin or prostaglandin-like compound; and

(b) a safe and potentiable amount of a histamine-2 receptor blocking anti-secretory agent;

wherein said gastric cytoprotective prostaglandin is administered at a level below which it unilaterally provides maximum intrinsic activity; and further wherein said histamine-2 receptor blocking anti-secretory agent is administered at a level below which it unilaterally provides maximum intrinsic activity.

The compositions and methods of the present invention are particularly useful against gastric inflammatory diseases which have not been induced by a non-steroidal anti-inflammatory agent.

The compositions of the present invention are particularly useful for oral administration.

Preferred prostaglandins or prostaglandin-like compounds useful in the compositions and methods of the present invention include

$PGE_1$;

$PGE_2$;

15,15-dimethyl $PGE_2$;
methyl-(S)-15-hydroxy-7-oxa-9-oxo-prost-13,14-ynoate;
15-hydroxy-7-oxa-9-oxo-prost-13,14-ynoic acid
methyl-15-oxo-7-oxa-9-oxo-prost-13,14-ynoate;
15-oxo-7-oxa-9-oxo-prost-13,14-ynoic acid;
[the 7-oxa-11-deoxy-13,14-didehydro-15-oxo analogue of $PGE_1$];
the 11-deoxy-13,14-didehydro-15-oxo analogue of $PGE_1$;
the 11-deoxy-13,14-didehydro-15-oxo analogue of $PGE_2$;
$PGA_1$;
$PGB_1$;
$PGF_{2\alpha}$;
$PGF_{2\beta}$; and
$PGD_1$; as well as pharmaceutically-acceptable salts, esters and mixtures thereof.

Preferred histamine-2 receptor blocking anti-secretory agents to be employed in the compositions and methods of the present invention include cimetidine, ranitidine, and mixtures thereof, with cimetidine being most preferred.

Preferred levels to be employed in the compositions and methods of the present invention of gastric cytoprotective prostaglandin or prostaglandin-like compounds are in the range of from about 0.25% to about 50% of the amount of gastric cytoprotective prostaglandin compound which unilaterally demonstrates maximum intrinsic activity. Preferred levels to be employed in the compositions and methods of the present invention of the histamine-2 receptor blocking anti-secretory agent are in the range of from about 1% to about 50% of the amount of anti-secretory agent which unilaterally demonstrates maximum intrinsic activity.

## DETAILED DESCRIPTION OF THE INVENTION

Prostaglandins (PGs) are generally considered a group of 20 carbon oxygenated fatty acids that is present in most mammalian cells and tissues. Approximately 20 of these chemicals have been identified in tissues and body fluids, whereas many hundreds

have been synthesized as analogues or derivatives of the naturally occurring PGs. In 1975 it was reported for the first time that included among the many physiological functions of PGs is a property referred to as "cytoprotection". Robert, A., Anti-Secretory, Anti-Ulcer, Cytoprotective and Diarrheogenic Properties of Prostaglandins, Advances in Prostaglandin and Thromboxane Research, Raven Press, New York, p. 507 (1975). Cytoprotection is generally defined as the ability to protect or insulate cells from chemically-induced damage or impairment of function. It is the prostaglandins which possess this cytoprotective property which are useful in the present invention. More specifically, it is the prostaglandins which possess the ability to protect the stomach and intestinal tract cells which are useful herein.

The present invention requires the administration of a sub- or non-anti-secretory level of a gastric cytoprotective prostaglandin or prostaglandin-like compound which is effective in the treatment and prevention of gastric inflammatory disease. The level of administration required for the prostaglandin in the present invention is a level sufficient to trigger a potentiation of the histamine-2 receptor blocking anti-secretory agent, but significantly less than the level which demonstrates maximum intrinsic activity as a cytoprotective agent in the model used to select the prostaglandin. "Maximum" or "maximal intrinsic activity" is recognized in the art to define the highest degree of protection an agent is capable of providing as a cytoprotective agent when administered by itself. This is the point where even substantial increases in dosage levels do not afford more protection in the model one has chosen to test the prostaglandin. For example, assume prostaglandin X is determined to be a candidate for use in the present invention. It has been shown to be cytoprotective in the ethanol necrosis model. It is further demonstrated that 1.0 mg/kg demonstrates 100% protection against the alcohol. 0.9 mg/kg, while demonstrating cytoprotective activity, does not provide 100% protection. Further, 1.1 mg/kg would not be useful because more than 100% protection cannot be

provided, and an increase in dosage will only increase the risk of side effects. Accordingly, 1.0 mg/kg is the lowest or minimum level or dosage which demonstrates maximum intrinsic activity when the prostaglandin is used as a cytoprotective agent. This would be the starting point to calculate levels useful in the present invention. In the practice of the present invention, preferred levels of prostaglandin X would then be 0.0025 mg/kg [0.25% of 1.0 mg/kg] to about 0.5 mg/kg [50% of 1.0 mg/kg].

For the purposes of the present invention, gastric cytoprotective prostaglandins are preferably selected from among those prostaglandins, or prostaglandin-like compounds, which, when administered orally to the rat, are capable of completely inhibiting the formation of ethanol-induced gastric lesions, chemical induced damage, or mechanically induced damage. Those which are shown to be cytoprotective in either or both of the following models are useful. Those which are found to be protective in Model 1 are particularly useful.

Model 1 - ETHANOL NECROSIS MODEL FOR CYTOPROTECTION

Female rats weighing approximately 200-220 g are placed in cages capable of preventing coprophagy and the eating of hair. After fasting overnight, 1.0 ml of 80% ethanol is administered orally to each rat. Test animals are treated with the gastric cytoprotective prostaglandin candidate orally in 1.0 ml of saline approximately one hour prior to the administration of ethanol. Similar animals used as controls are dosed only with the saline vehicle. One hour after the ethanol dosing, the animals are sacrificed and the stomach dissected out, being opened along the greater curvature, and are placed on white cards so that they may be examined for lesions or ulcers present in the corpus of the stomach under a dissecting microscope. The incidence of ulcers is recorded, the length of the ulcer may be measured and the severity graded on a scale of from 0 to 3 (0 being mild and 3 being the most severe). An averaged numerical score is then determined of severity and the average number of ulcers per stomach determined. From these values the ulcer index is then calculated by adding (a) 10% of the incidence of animals with

ulcers, (b) the mean severity of the ulcers present, and (c) the average number of ulcers per stomach. The lower the score, the better the protection. The maximal intrinsic activity for the agent is the lowest score (lowest ulcer index) the agent is capable of achieving in the model. The smallest dose of the agent which yields this lowest ulcer index is the lowest dosage or level of administration which unilaterally demonstrates maximal intrinsic activity.

A second method for determining whether a prostaglandin or prostaglandin-like compound is a gastric cytoprotective prostaglandin is as follows. The following model is also useful in determining whether a histamine-2 receptor blocking anti-secretory agent is useful in the present invention.

Model 2 - CYSTEAMINE DUODENAL ULCER MODEL

Male Sprague-Dawley rats weighing between 180 and 230 g are fasted for 24 hours during which time they are given *ad libitum* access to tap water. At the end of the 24 hour fast period, 500 mg/kg of cysteamine is administered subcutaneously. At the same time, the drug or control treatment is begun. The drug or control treatment consists of administering the drug or vehicle/control at 24 hours, 28 hours, and 32 hours after the fast has begun. That is, the drug, or vehicle/control treatment is administered at 0, 4 and 8 hours after the cysteamine. 48 hours after the fasting has begun, 24 hours after the administration of cysteamine, the rats are anesthetized and sacrificed.

After sacrifice, the abdominal cavity is opened, and the stomach and approximately 2 inches of duodenum are excised and removed from the rat. They are placed on a 4" x 4" labelled index card. Care must be taken not to disturb the duodenum as this could affect the grading. The stomach and duodenum are cut open along the greater curvature and longitudinally along the anti-mesenteric side, respectively. The duodenum is carefully spread out without altering the orientation of the lesions and the thickness of the mucosa. They are viewed through a stereo microscope at approximately 7x magnification for gross examination of the stomach, pylorus and duodenal surface.

For each duodenum, the amount of erythema, the amount of edema, a total lesion area and the severity of the lesions are evaluated according to the grading scale in Table 1.

This is also repeated for the stomach. This information is recorded.

The lower the score, the greater the protection. The lowest score which an agent can achieve in this model is the maximal intrinsic activity for this agent in this model. The smallest dosage of this agent which achieves this lowest score is the lowest dosage or level of administration which yields maximal intrinsic activity for this agent.

| | Very Small | Small | Small to Moderate | Moderate | Moderate to Large | Large | Very Large |
|---|---|---|---|---|---|---|---|
| Erosion | 1 | 1.5 | 2 | 2.5 | 3 | 3.5 | 4 |
| Surface Lesion | 3 | 4 | 5 | 6 | 7 | 8 | 9 |
| Deep Lesion | 8 | 9 | 10 | 11 | 12 | 13 | 14 |
| Perforated Ulcer | 13 | 14 | 15 | 16 | 17 | 18 | 19 |

Explanation for Table;

Erosion: An erosion exists if only the villi are affected. They may appear slightly different in texture and/or color. An erosion is present only if the lesion is not detectable from the serosal surface.

Surface Lesion: A surface lesion exists where there is site penetration into the mucosal layer. Generally, the villi are absent and there may be some clotted blood. Surface lesions are generally very mild lesions and they are seldom detectable from the serosal surface.

Deep Lesion: These are moderate to severe penetrations into the mucosal layers. They contain clotted or fresh blood and are usually always visible on the serosal surface. They often appear as dark raised areas.

Perforated Lesion: A perforated lesion is any lesion that has penetrated through the serosal surface.

With respect to the size of the lesion, i.e., very small to very large, this is an evaluation which is made by evenly grouping the actual size lesions present in this model. The smallest lesion detectable would fall in the class of very small, whereas the largest lesion would be classified as very large. The remainder are classified in between.

As will be appreciated from the above, gastric cytoprotective prostaglandins or prostaglandin-like compounds useful in the present invention are determined in accordance with the above criteria simply and efficiently, by readily available procedures, in a standard laboratory animal. As will also be appreciated from the above, histamine-2 receptor blocking anti-secretory agents useful in the present invention are determined in accordance with the above criteria simply and efficiently, by readily available procedures, in standard laboratory animals.

Further, both of the above models may be used to determine the lowest dosage or level of administration which exhibits maximal intrinsic activity for a prostaglandin or histamine-2 antagonist.

In the determination of the usefulness in the present invention and lowest dosage which exhibits maximal intrinsic activity as a cytoprotective agent, the alcohol necrosis model is preferred for prostaglandins, and (as an anti-secretory agent) the cysteamine model is preferred for histamine-2 receptor blocking anti-secretory agents.

The prostaglandins useful in the present invention include:

$PGE_1$;
$PGE_2$;
15,15-dimethyl $PGE_2$, methyl ester;
15,15-dimethyl $PGE_2$
$PGA_1$;
$PGB_1$;
$PGF_{2\alpha}$ ;
$PGF_{2\beta}$ ;
$PGD_1$;
15-methyl-$PGF_{2\beta}$ ;
15-methyl-$PGF_{1\beta}$ ;

15-methyl-$PGF_{2\beta}$, methyl ester;
16,16-dimethyl-$PGE_2$;
15-methyl-$PGF_{2\beta}$, methyl ester;
15-methyl-$PGE_2$, methyl ester;
15-epi-15-methyl-$PGE_2$, methyl ester;
15-epi-15-methyl-$PGF_{2\alpha}$, methyl ester;
15-dehydro-$PGF_{2\alpha}$;
17-phenyl-18,19,20-trinor-$PGF_{2\alpha}$;
$PGA_2$, methyl ester;
15-epi-15-methyl-$PGF_{2\alpha}$, methyl ester;
cis-4,5-didehydro-$PGF_{1\alpha}$;
16,16-dimethyl-$PGA_2$;
15-methyl-$PGA_2$, methyl ester;
17-(p-chlorophenyl)-18,19,20-trinor-$PGE_2$, methyl ester, 15-methyl ether;
15-dehydro-$PGE_2$;
$8\beta$,$12\alpha$-$PGF_{2\alpha}$, methyl ester;
$8\beta$,$12\alpha$-$PGF_{2\beta}$, methyl ester;
2a,2b-dihomo-17-phenyl-18,19,20-trinor-$PGE_2$, methyl ester, 15-methyl ether;
2a,2b-dihomo-17-phenyl-18,19,20-tetranor-$PGF_{2\alpha}$, methyl ester, 15-methyl ether;
15-epi-15-methyl-$PGF_{2\alpha}$, methyl ester;
$PGF_{2\alpha}$, 15-methyl ether;
15-methyl-$PGE_1$, methyl ester;
15-epi-15-methyl-11-deoxy-$PGF_{1\alpha}$, methyl ester;
15-epi-15-methyl-11-deoxy-$PGF_{2\beta}$, methyl ester;
16,16-dimethyl-$8\beta$, $12\alpha$-$PGE_2$ methyl ester;
11-deoxy-$PGE_1$, methyl ester, 15-methyl ether;
11-deoxy-$PGE_2$, methyl ester, 15-methyl ether;
11-deoxy-$PGF_{1\beta}$, methyl ester, 15-methyl ether;
15-epi-17-phenyl-18,19,20-trinor-$8\beta$, $12\alpha$-$PGE_2$, methyl ester;
9,11-dideoxy-$9\alpha$,$11\alpha$-epoxymethano-$PGF_1$, methyl ester;
15-epi-15-methyl-$PGF_{2\alpha}$, methyl ester;
$8\beta$, $12\alpha$-$PGE_2$, methyl ester;

$PGD_2$;
5-oxa-$PGE_1$, methyl ester;
15-epi-5-oxa-8β, 12α-$PGA_1$;
$PGF_{1\alpha}$, methyl ester, 15-methyl ether;
$PGE_1$, methyl ester;
15-dehydro-cis-4,5-didehydro-$PGF_{1\alpha}$, methyl ester;
15-epi-15,16,16-trimethyl-$PGF_{2\alpha}$, methyl ester;
2a,2b-dihomo-$PGE_2$, methyl ester;
15-epi-16-phenoxy-17,18.19,20-tetranor-8β,12α-$PGF_{2\alpha}$, methyl ester;
16-phenoxy-17,18,19,20-tetranor-8β,12α-$PGF_{2\beta}$, methyl ester;
15-epi-15,16,16-trimethyl-$PGE_2$, methyl ester;
15-epi-15-methyl-$PGD_2$, methyl ester;
15-methyl-$PGD_2$, methyl ester;
8β, 12α-$PGE_2$;
9-deoxy-9,10-didehydro-15-methyl-$PGD_2$, methyl ester;
9-deoxy-9-hydroxymethyl-$PGF_2$ ;
cis-4,5-didehydro-$PGD_1$, methyl ester;
16,16-dimethyl-$PGA_2$, methyl ester;
$PGF_{2\alpha}$, 1,15-lactone;
$PGD_2$, methyl ester;
15-epi-2a,2b-dihomo-15-methyl-$PGF_{2\alpha}$, methyl ester;
2a,2b-dihomo-15-epi-15-methyl-$PGF_{2\alpha}$, methyl ester;
cis-4,5-didehydro-$PGE_1$;
cis-4,5-didehydro-$PGD_1$;
cis-4,5-didehydro-$PGA_1$;
2a,2b-dihomo-16,16-dimethyl-$PGF_{2\alpha}$;
2a,2b-dihomo-16,16-dimethyl-$PGF_{1\alpha}$, methyl ester;
9-deoxy-9,10-didehydro-$PGD_2$;
15-methyl-$PGF_{2\beta}$, methyl ester;
15-epi-15-methyl-$PGF_{2\beta}$;
16,16-dimethyl-cis-13-$PGE_2$;
15-epi-cis-4,5-didehydro-$PGF_{1\alpha}$;
15-epi-cis-4,5-didehydro-$PGF_{1\beta}$;
15-epi-cis-4,5-didehydro-$PGD_1$;
2,2-difluoro-15-methyl-$PGE_2$, methyl ester;

5-oxa-16,16-difluoro-$PGE_1$, methyl ester;
15-epi-15-methyl-$PGF_{2\alpha}$, 1,9-lactone;
16,16-difluoro-$PGE_2$;
cis-13-$PGE_1$;
15-epi-15-methyl-$PGF_{2\beta}$, 1,9-lactone;
5,6-didehydro-16,16-dimethyl-$PGE_2$;
13,14-didehydro-$PGF_{1\beta}$;
13,14-didehydro-$PGF_{1\alpha}$, 1,15-lactone;
$PGE_3$, methyl ester;
3,7-inter-m-phenylene-4,5,6-trinor-3-oxa-$PGE_1$;
17-(o-methoxyphenyl)-18,19,20-trinor-15-epi-$PGE_2$;
8 , 12 -13,14-didehydro-$PGF_{2\alpha}$;
13,14-didehydro-15-epi-$PGE_2$;
13,14-didehydro-15-epi-$PGE_1$;
9-deoxy-9-methylene-$PGF_2$;
13,14-didehydro-$PGF_{1\alpha}$, 1,9-lactone;
15-epi-15-methyl-9-deoxy-9-methylene-$PGF_2$;
15-methyl-9-deoxy-9-methylene-$PGF_2$;
15-epi-15-methyl-$PGE_1$;
13,14-didehydro-8β,11β,12 -$PGE_1$;
15-epi-15-methyl-9-deoxy-9,10-didehydro-$PGD_2$;
16,16-dimethyl-$PGE_2$, p-benzamidophenyl ester;
$PGE_2$, ethyl amide; and
16,16-dimethyl-$PGF_{2\alpha}$.
(+)(16RS)-15-deoxy-16-hydroxy-16-methyl-$PGE_1$, methyl ester;
11-deoxy-11-alpha-16,16-trimethyl-$PGE_2$;
(+)-11-alpha,16 alpha, beta-dihydroxy-1,9-dioxo-1-(hydroxy methyl)-16-methyl-13-trans-prostene
prostacylines, esp. $PGI_2Na$
$PGE_1$- α -cyclodextrin Clathrate
(+)-4,5,-didehydro-16-phenoxy-17,18,19,20-tetranor-$PGE_2$, methyl ester
16,16-dimethyl-4,5-cis-didehydro-16- m -chlorophenoxy-17,18,19,20-tetranorprostaglandin $E_1$, methyl ester
15-methyl-4,5- cis -didehydro-16- m-chlorophynoxy-17,18,19,20-tetranor-prostaglandin $E_1$, methyl ester

16,16-dimethyl,16- m-chlorophenoxy-17,18,19,20-tetranor-prostaglandin $E_2$

7-oxa-11-deoxy prostaglandin analogues useful in the present invention are of the formula

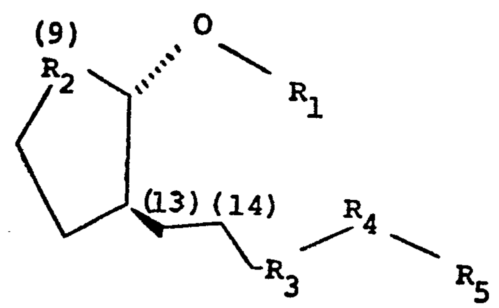

wherein the $C_{13}$-$C_{14}$ bond is a single bond, cis double bond, or trans double bond, or triple bond; $R_1$ is

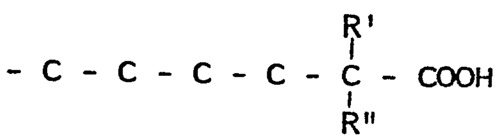 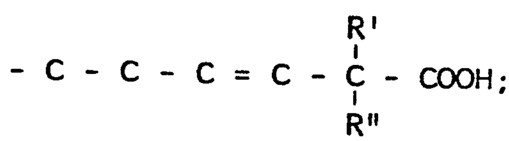

or

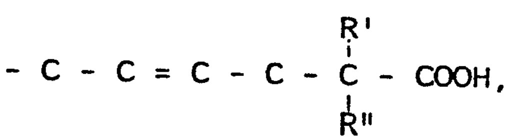 wherein

R' and R" are each H, $CH_3$, $C_2H_5$ or $C_3H_7$;

$R_2$ is C = O or 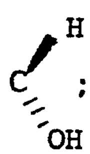 ; $R_3$ is $CH_2$,

C = O, 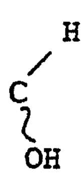 , 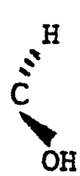 , 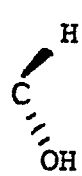 , 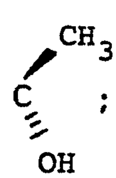 ;

$R_4$ is $CH_2$, C = O, 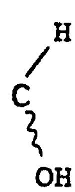 , 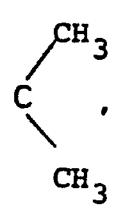 , 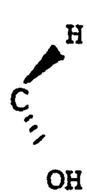 , 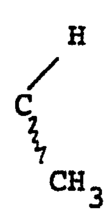 or 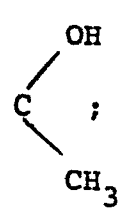 ;

$R_5$ is $C_2H_5$ or $C_4H_9$;
and esters and salts thereof.

7-oxa-11-deoxy prostaglandin analogues useful in the present invention are also of the formula

(9)

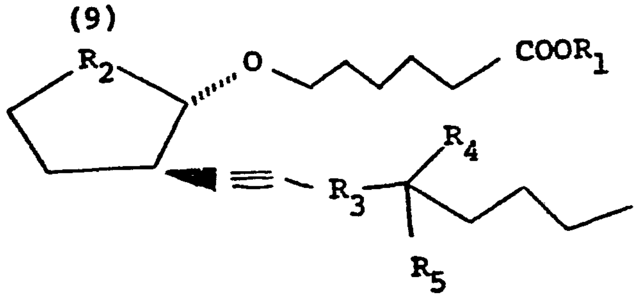

wherein $R_1$ is H or $CH_3$; $R_2$ is C = O or 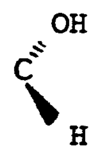 ;

$R_3$ is 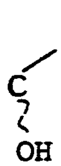 , 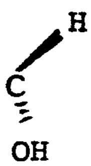 , 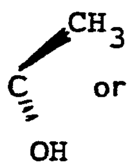 or 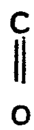; $R_4$ and $R_5$ are each H or $CH_3$. See U.S. Patent 4,345,984, Mihelich, issued August 24, 1982, expressly incorporated herein by reference for the synthesis of these analogues.

Prostaglandin analogues useful in the present invention also include 13,14 didehydro-15-oxo-prostaglandin analogues of the general formula:

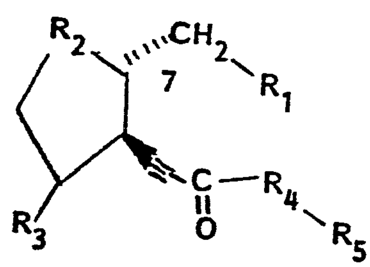

wherein $R_1$ is

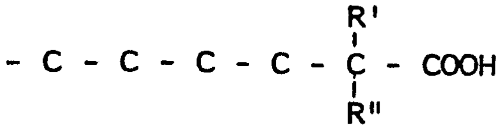 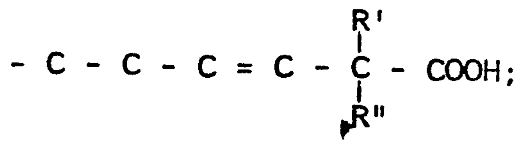

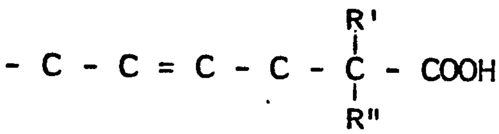 or

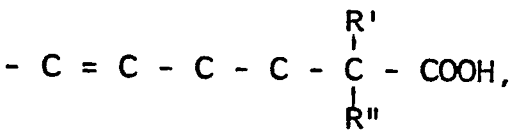 and pharmaceutically-acceptable salts and esters thereof, wherein R' and R" are each independently H, $CH_3$, $C_2H_5$ or $C_3H_7$;

$R_2$ is 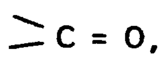 , 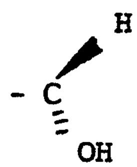 , 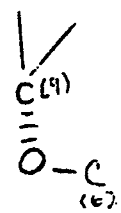 or 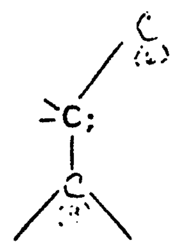

$R_3$ is hydrogen, hydroxyl, or hydroxymethyl;

$R_4$ is $CH_2$, 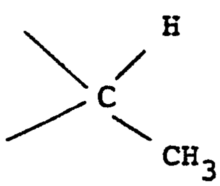 or 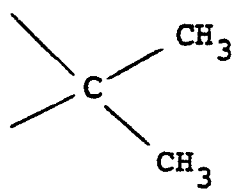 ; and

$R_5$ is $C_2H_5$, $C_3H_7$, $C_4H_9$ or $C_5H_{11}$.

Of these, particularly preferred are the prostaglandin analogues of the formula:

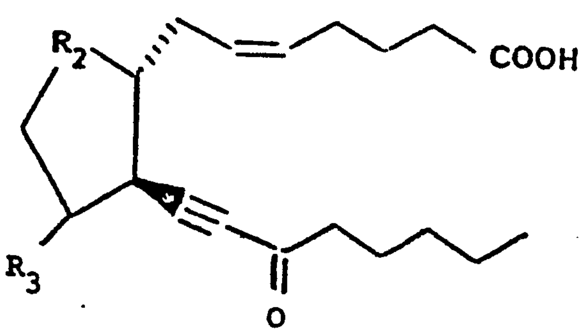

wherein $R_2$ is 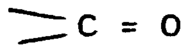 or 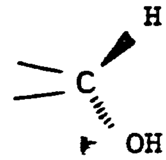 ; and

$R_3$ is hydrogen, hydroxyl, or hydroxymethyl; and salts and esters thereof. See U.S. Patent Application Serial No. ____________, filed November 5, 1982, "Novel Prostaglandin Analogues", Randall S. Matthews, expressly incorporated herein by reference.

Mixtures of all the above groups of prostaglandins may also be employed.

As has been stated above, a number of gastric cytoprotective prostaglandins or prostaglandin-like compounds useful in the present invention also exhibit an anti-secretory effect. In general, in order to be anti-secretory, the prostaglandin or prostaglandin-like compound must be administered at a substantially higher dosage level than the level necessary to be cytoprotective, which in turn, is much higher than the level necessary in the practice of this invention. Therefore, in accordance with the present invention, the prostaglandin or prostaglandin-like compounds which are preferred in the present invention are those which are active in either of the rat cytoprotective models, but which exhibit a significant anti-secretory effect, if at all, when administered (in the rat) at a level which is 10 times, and preferably 100 times, greater than the level at which the compound exhibits maximum intrinsic activity. Experimental methods are commonly available for the determination of comparing the gastric anti-secretory activity with the gastric cytoprotective activity. For example, those described by Robert, et al., Gastroenterology 55: 481 (1968) and 70: 359 (1976) are easily and conveniently employed. See also U.S. Patent 4,097,603, Robert, issued June 27, 1978, expressly incorporated herein by reference.

Among the gastric cytoprotective prostaglandins preferred for use in the instant invention are those which have no capability of significantly inhibiting gastric secretions at any level of administation. The synthesis of these compounds are well-known, and reference may be had to a standard text such as Bindra, et al., Prostaglandin Synthesis, Academic Press (1977).

The gastric cytoprotective prostaglandins or prostaglandin-like compounds preferred in the instant invention include:

$PGE_1$;

$PGE_2$;

15,15-dimethyl $PGE_2$;

15,15-dimethyl $PGE_2$, methyl ester;

methyl-(S)-15-hydroxy-7-oxa-9-oxo-prost-13,14-ynoate;

15-hydroxy-7-oxa-9-oxo-prost-13,14-ynoic acid;

methyl-15-oxo-7-oxa-9-oxo-prost-13,14-ynoate;

15-oxo-7-oxa-9-oxo-prost-13,14-ynoic acid

the 11-deoxy-13,14-dihydro-15-oxo analogue of $PGE_1$;

the 11-deoxy-13,14-dihydro-15-oxo analogue of $PGE_2$;

$PGA_1$;

$PGB_1$;

$PGF_{2\alpha}$;

$PGF_{2\beta}$; and

$PGD_1$, as well as pharmaceutically-acceptable salts and esters, and mixtures thereof.

Of these, the 11-deoxy-13,14-dihydro-15-oxo analogue of $PGE_1$ is most highly preferred.

Within the practice of the present invention, it is possible to employ the gastric cytoprotective prostaglandins, as identified by the models described above, or any other convenient model for identifying a cytoprotective prostaglandin, at below the smallest levels which they would ordinarily exhibit maximal intrinsic activity as cytoprotective agents against the gastric inflammatory diseases if they were administered in the absence of the histamine-2 receptor blocking anti-secretory agent. For example, when the prostaglandin or prostaglandin-like compound is identified as being a gastric cytoprotective compound in the alcohol model, one can simultaneously determine the lowest dosage or level of administration which demonstrates maximal cytoprotective intrinsic activity, i.e., the minimum dosage which gives the maximum benefit. This level of prostaglandin, the

lowest level which can be administered in a particular model which achieves the maximal cytoprotective intrinsic activity, (the most efficacy) is, in conventional practice, the most desirable level to administer. It provides the highest degree of efficacy and the lowest level of side effects. However, within the practice of the present invention, levels below these may be employed very effectively. For example, the physician may administer a level of about 0.25% to about 50% of the lowest level which has been determined to exhibit the maximal cytoprotective intrinsic activity. Preferred levels of gastric cytoprotective prostaglandin are levels of about 10% to about 25% of the maximal intrinsic activity. However, it will be appreciated that levels below 10% of the amount usually required to demonstrate maximum intrinsic activity may be employed where the potentiation effect of the present invention is particularly marked.

In practicing a preferred embodiment of this invention, the physician would begin by administering the gastric cytoprotective prostaglandin at a level of about 50% of the minimum level ordinarily required to exhibit maximal intrinsic activity concurrently with a level of histamine-2 receptor blocking anti-secretory agent as described and preferred herein. Thereafter, the level of administration of the gastric cytoprotective prostaglandin would be lowered in accordance with the patient's response. Once the minimum potentiable or potentiating dose of the gastric cytoprotective prostaglandin is determined for the specific patient (in combination with the histamine-2 receptor blocking anti-secretory agent), the patient is advantageously provided with a treatment regimen which will provide a substantially uniform and effective level of protection throughout the course of the therapy employing the lowest dosage rate which will provide this level of prostaglandin to further reduce side effects, costs and the like.

Within the method of the present invention, any convenient systemic route of administration for the cytoprotective prostaglandin may be employed. See U.S. Patent 3,903,297, Robert, issued September 2, 1975, expressly incorporated herein by reference,

for a complete description of the methods by which prostaglandins may be formulated, and the routes by which they may be administered, within the practice of the present invention. Oral administration is the most highly preferred route.

In the practice of the method of the present invention, the gastric cytoprotective prostaglandin compound is administered concurrently or simultaneously (as defined herein) with a histamine-2 receptor blocking anti-secretory agent. The histamine-2 receptor blocking anti-secretory agents, or $H_2$-receptor antagonists, useful in the present invention include cimetidine, ranitidine, burimamide, metiamide, tiotidine, and oxmetidine as well as compounds of the formula:

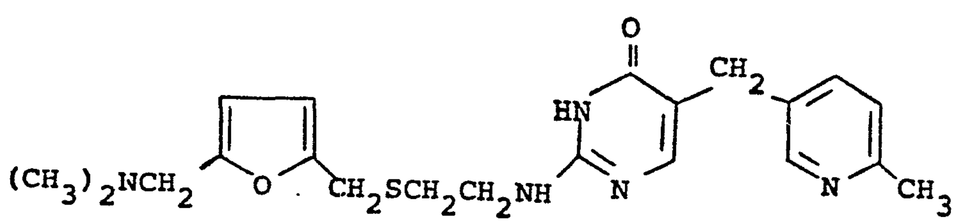

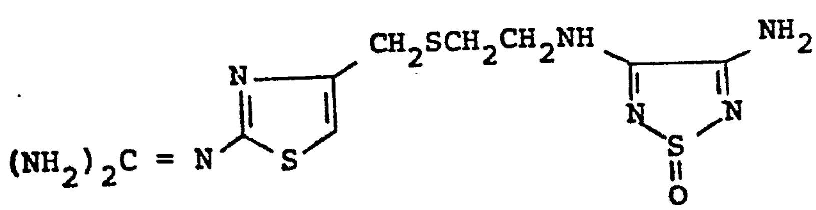

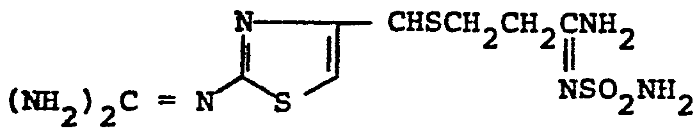

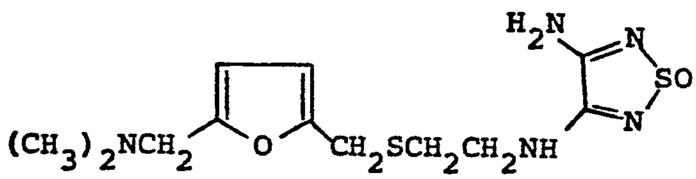

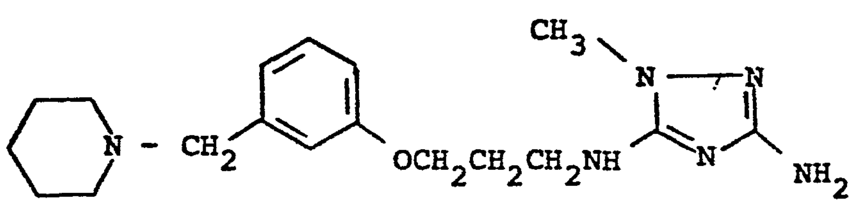

The above structures are well-known in the art. See C&E News, April 12, 1982, pp 24-26, expressly incorporated herein by reference. Mixtures of the above histamine-2 receptor blocking anti-secretory agents may also be employed.

The most preferred of these compounds are cimetidine, ranitidine, and mixtures thereof, with cimetidine being especially preferred.

In the operation of the present invention, any convenient systemic route of administration for the histamine-2 receptor blocking anti-secretory agent may be employed. However, oral administration and subcutaneous administration are particularly preferred. Oral administration is most highly preferred.

While histamine-2 receptor blocking anti-secretory agents, such as cimetidine, have been shown to be effective in the treatment of the bleeding and pain associated with acute gastrointestinal and gastroduodenal mucosal lesions in man, endoscopic evaluations indicate that during treatment with such compounds, the incidence of gastric inflammatory lesions is still significant. Halloran, et al., "Prevention of Acute Gastrointestinal Complications After Head Injury; A Controlled Study of Cimetidine Prophylaxis." American Journal of Surgery 139: 44 (1980). Accordingly, within the use of the present invention, the maximal intrinsic activity of a histamine-2 receptor blocking anti-secretory agent, unlike the maximum or maximal intrinsic activity of a prostaglandin, is less than complete protection against gastric damage. Thus, by the term "maximum or maximal intrinsic activity of a histamine-2 receptor blocking anti-secretory agent" is meant the maximum amount of protection, or maximum reduction in damage, as can be demonstrated by the anti-secretory agent. If a reduction in damage of 70%, rather than 100%, is the most activity a given anti-secretory agent can demonstrate, then this would be the maximum intrinsic activity.

In practicing the methods of the present invention, a level of histamine-2 receptor blocking anti-secretory agent of about 1% to about 50% of the lowest dosage which conventionally yield the maximal intrinsic activity as an anti-secretory agent may be employed. Preferred levels are dosages in the range of about 5% to about 50%, and more preferably 5% to about 35% of the lowest level which conventionally yields the maximal intrinsic activity. All these levels are significantly below the amount of histamine-2 receptor blocking anti-secretory agent which would have to be

employed in the absence of the present invention in order to achieve significant clinical efficacy or maximize the protection afforded by the anti-secretory agent if it were to be administered alone.

In the treatment of gastric inflammatory disease, in an average 65 kilo man, an oral dosage of 300 mg of cimetidine 4 times a day has been demonstrated to be clearly superior to a placebo (but no better than intensive antacid therapy) in promoting healing. (However, cimetidine has still been the preferred method of treatment because of the inability of patients to tolerate adequate therapeutic doses of antacids as a result of their lack of palatability and the resulting bowel disturbances.) In the practice of the present invention it is possible to employ dosage levels of cimetidine which are significantly lower than these amounts. Accordingly, in the practice of the present invention, dosage levels of 1.5 mg to about 150 mg per dose of cimetidine in an average 65 kilo man may be employed. Preferred levels of cimetidine are dosages of about 15 to about 150 mg, and, most preferably, about 30 to about 150 mg of cimetidine per dose are administered concurrently, as defined and preferred herein, with the gastric cytoprotective prostaglandins, as described and preferred herein. Dosage levels of cimetidine of about 30 mg to about 100 mg are most highly preferred. The above dosages may be given to a 65 kilo man from one to about four times per day. In cases where the gastric inflammatory disease has led to hemmorage, cimetidine has been used in doses of about 300 mg intravenously every 4 hours. In the practice of the present invention, these dosage levels for intravenous administration may be significantly reduced. Dosage levels of about 15 to about 150 mg intravenously are preferred when subcutaneous or intravenous methods of administration are elected. The most preferred method of administration for the cimetidine within the practice of the present invention is the oral route of administration. In general, however, these dosage levels will be independent of body weight. Should body weight affect the efficacy of the cimetidine, the above dosages can be used to determine the pharmaceutically

equivalent dosage by using these amounts as being administered to a 65 kilogram man.

In practicing the method of the present invention, the prostaglandin compound and anti-secretory agent, as defined and preferred above, are administered either concurrently, i.e., with the first agent being administered within no more than about 24 hours, preferably no more than about 4 hours, and most preferably, no more than about 2 hours, prior to the administration of the agent selected to be administered second. In the most highly preferred method of treatment of the present invention, the prostaglandin and anti-secretory agent, as defined and preferred above, are administered simultaneously, in any order, within 0 to about 2 minutes of each other. It will also be appreciated that, if the 2 components are not administered simultaneously, the prostaglandin is preferably administered first. However, the potentiation of the present invention can take place with the components being administered in any sequence over a period of up to about 24 hours.

The treatment regimen and duration for the administration of the gastric cytoprotective prostaglandin/anti-secretory agent combination of this invention will depend on a wide variety of factors. The dosage level and duration of treatment will vary, within the scope of sound medical judgement, with the age and physical condition of the patient, the type of patient being treated, the patient's history of gastric inflammatory disease, the projected duration of the treatment at the outset, the specific prostaglandin or prostaglandin-like compound being employed, the specific histamine-2 receptor blocking anti-secretory agent being employed, other therapies being employed, as well as other factors within a specific knowledge of the patient by the physician.

Should the specific patient be more tolerant of the gastric cytoprotective prostaglandin than the histamine-2 receptor blocking antagonist, the physician would then begin the treatment regimen at a gastric cytoprotective prostaglandin level of administration of about 50% of the lowest level found to demonstrate

maximal intrinsic activity, in combination with a level of histamine-2 receptor blocking anti-secretory agent of about 150 mg. Thereafter, the physician would begin to reduce the dosage level of the histamine-2 receptor blocking anti-secretory agent until the minimum potentiating or potentiable amount of anti-secretory agent is established. Thereafter, this patient would advantageously be provided with a dosage regimen which would provide a substantially uniform level of protection throughout the course of therapy at the lowest possible level of administration of anti-secretory agent.

The treatment of gastric inflammatory disease with the gastric cytoprotective prostaglandin combination histamine-2 receptor blocking anti-secretory agent is preferably continued so long as the patient remains at risk to the gastric inflammatory disease. Accordingly, the treatment may last for a period of 4-6 weeks, or may be continued indefinitely in a patient who has demonstrated a history of recurrent gastric or duodenal ulceration.

The compositions of the present invention may be formulated in any standard dosage forms; however, the invention is particularly beneficial when it is formulated for oral ingestion, for example, in the form of tablets, capsules, lozenges, liquids, emulsions or pills. In producing a unit dosage form of the compositions of the present invention, each dosage contains from about 0.25% to about 50% of the level of gastric cytoprotective prostaglandin which unilaterally demonstrates maximal cytoprotective intrinsic activity and about 1% to about 50% of the lowest dosage of histamine-2 receptor blocking anti-secretory agent which yields the maximal intrinsic activity. The dosage forms may, in addition to the required components, contain additional compatible pharmaceutically-active materials in safe and effective amounts, as well as adjunct formulational-type components conventionally used in the preparations of pharmaceutical formulations, such as, carriers, excipients, anti-oxidants, tablet disintegrating agents, preservatives,

colorings, flavors, stabilizers, fillers, and diluents, at their art-established usage levels.

By the term "maximum or maximal intrinsic activity" is meant the highest degree of protection from lesions, ulceration and inflammation which an agent is conventionally capable of providing when unilaterally administered in the model selected to determine its usefulness in the present invention. With respect to prostaglandins, it is the highest degree of protection it is conventionally capable of providing used as a cytoprotective agent. With respect to the histamine-2 receptor blocking anti-secretory agents, it is the highest degree of protection the agent can provide as a conventional anti-secretory agent.

By the term "unilateral", as used herein, is meant that the effects of this agent are analyzed in the absence of any other therapy.

By the term "lowest level which unilaterally demonstrates maximal intrinsic activity" of an agent, as used herein, is meant, the smallest dose or smallest level of administration of that agent which provides the maximum amount of protection, or maximum reduction in damage, in the model which is used to determine the potential capability of the agent for being useful within the practice of the present invention. In the case of any conflict between models, i.e., if the agent is shown to be useful in more than one model, the suitability of any prostaglandin will be measured in the alcohol necrosis model. In the event of any conflict between models, i.e., conflicting maximal intrinsic levels are determined, the lowest level which demonstrates maximal intrinsic activity for a prostaglandin or prostaglandin-like compound will also be measured in the alcohol necrosis model. In the event of any conflict between models, i.e., if the agent is shown to be useful in more than one model, the suitability of a histamine-2 receptor blocking anti-secretory agent as a candidate will be determined in the cysteamine model. Further, in the event of any conflict, i.e., conflicting maximal intrinsic levels are determined,, the determination of the lowest level which demonstrates maximal intrinsic activity for a histamine-2 receptor blocking

anti-secretory agent will also be determined in the cysteamine model.

By "pharmaceutically-acceptable", as used herein, is meant that the drug compounds and other ingredients used in the present compositions and processes are suitable for use in contact with the tissues of human and lower animals without undue toxicity, irritation, allergic response and the like commensurate with a reasonable benefit/risk ratio.

The term "administration" of the composition herein includes any method which will provide systemic use, for example, as by injection, intravenous infusion, suppositories, and the like. This term will preferably include oral or intravenous infusion of histamine-2 receptor blocking anti-secretory agents, and will preferably mean oral administration of the prostaglandin or prostaglandin-like compound.

By the term "comprising", as used herein, is meant that various additional compatible drugs and medicaments, as well as inert ingredients, can be conjointly employed in the compositions and processes of this invention, as long as the critical prostaglandin and histamine-2 receptor blocking anti-secretory agents are used in the manner disclosed.

By "compatible", as used herein, is meant that the components of the compositions are capable of being comingled without interacting in a manner which would substantially decrease the efficacy of the total compositions and methods under ordinary use situations.

By "carrier", as used herein, is meant a liquid, fluid or solid material which can optionally be used to provide finished compositions for use in the present invention for systemic, oral or other routes of administration.

By "person at risk", or "person in need of such treatment", as used herein, is meant those individuals who suffer a significant risk of ulceration, or other gastrointestinal damage or discomfort, if left untreated, or if the acid levels of the gut remain uncontrolled. For example, those whose gasto-intestinal mucosa has already been compromised or ulcerated; those who

have been diagnosed as suffering from chronic hyperchlorhydria; those suffering from peptic ulcer disease and its complications, such as hemmorage or penetration; those who have been diagnosed as having a pathology which in turn causes hyperchlorhydria, such as Zollinger-Ellison Syndrome and related diseases; those who have or will shortly undergo gastro-intestinal surgery, i.e., pre- and post-operative gastro-intestinal surgical patients; those suffering from shock or trauma; those undergoing other treatment regimens which can cause hypochlorhydria or gastric inflammatory disease except non-steroidal anti-inflammatory agents; those who have had recent acute exposure to a cytodestructive agent, such as ionizing electromagnetic or particular radiation; those who have had acute or chronic gastro-intestinal exposure, for example, by injestion, of noxious gastric cytodestructive or cytotoxic chemical agents; or those who are diagnosed as being exposed to a stressful environment regardless or origin, especially those exposed to this environment who have demonstrated recurrent gastritis or ulcer disease when exposed to such an environment.

By the term "potentiate", as used herein, is meant that when the level of protection of x grams of prostaglandin z and y grams of histamine-2 receptor blocking anti-secretory agent a are measured, and then compared to the level of protection afforded by the administration of combinations of these compounds within the scope of the present invention, the level of protection afforded by the combination will be more than the sum of the amount of protection offered by each agent when administered by itself, i.e., by its unilateral action. This "more than additive" effect is often referred to as synergism.

By the term "potentiating amount" of an agent is meant a sufficient amount of this agent to increase the action, activity or effect of a second agent.

By the term "potentiable amount" of an agent is meant a sufficient amount of that agent to have its action, activity or effect increased by a second agent.

The following non-limiting Examples demonstrate the methods and compositions of the present invention wherein prostaglandin or prostaglandin-like compounds are administered in combination with histamine-2 receptor blocking anti-secretory agents in the prophylaxis of gastric ulcers.

In Examples I-VIII, all were conducted in accordance with the cysteamine gastric ulcer model described above. The vehicle was 0.75% polyoxyethylene (20) sorbitan mono-oleate (Tween 80). All dosages were at a rate of 2.5 ml/kg. The treatment compositions were orally dosed at a volume of 2.5 ml/kg, in accordance with the model, at 24, 28 and 32 hours after the beginning of the fast, or 0, 4 and 8 hours after the subcutaneous administration of cysteamine. In preparing the prostaglandin + cymetidine compositions, the selected amount of prostaglandin and selected amount of cimetidine were added to a volume which was equivalent to 2.5 ml/kg for each animal. This was then given to the animal at 24, 28 and 32 hours respectively, after the beginning of the fast. The damage grading score was in accordance with the table provided in the cysteamine gastric ulcer model.

The dosage levels administered above are far below the level which exhibits maximal intrinsic activity for the prostaglandin as a cytoprotective agent or the cimetidine as an anti-secretory agent.

EXAMPLE I

| Group | No. | TREATMENT (PG) Compound | Dosage Level | RESULT Damage Grading Score | % of Cntrl. | % of Redc'n. |
|---|---|---|---|---|---|---|
| 1 | 12 | veh. cntrl. | 2.5 ml/kg (x3) | 9.0 ± 2.2 | 100 | 0 |
| 2 | 11 | PG-V | 5 mg/kg (x3) | 12.6 ± 2.6 | 140 | -40 |
| 3 | 11 | Cimetidine | 25 mg/kg (x3) | 12.0 ± 2.0 | 133 | -33 |
| 4 | 11 | PG-V + Cimetidine | 5 mg/kg + 25 mg/kg (x3) | 6.2 ± 1.6 | 69 | 31 |

EXAMPLE II

| Group | No. | TREATMENT (PG) Compound | Dosage Level | RESULT Damage Grading Score | % of Cntrl. | % of Redc'n. |
|---|---|---|---|---|---|---|
| 1 | 11 | veh. cntrl. | 2.5 ml/kg (x3) | 15.5 ± 4.5 | 100 | 0 |
| 2 | 11 | PG-V | 5 mg/kg (x3) | 16.4 ± 3.3 | 106 | -6 |
| 3 | 10 | Cimetidine | 25 mg/kg (x3) | 24.6 ± 1.3 | 159 | -59 |
| 4 | 11 | PG-V + Cimetidine | 5 mg/kg + 25 mg/kg (x3) | 10.0 ± 2.5 | 65 | 35 |

EXAMPLE III

| Group | No. | TREATMENT (PG) Compound | Dosage Level | RESULT Damage Grading Score | % of Cntrl. | % of Redc'n. |
|---|---|---|---|---|---|---|
| 1 | 11 | veh. cntrl. | 2.5 ml/kg (x3) | 18.3 ± 3.7 | 100 | 0 |
| 2 | 10 | PG-V | 5 mg/kg (x3) | 21.5 ± 4.6 | 117 | -17 |
| 3 | 9 | Cimetidine | 50 mg/kg (x3) | 11.1 ± 3.0 | 61 | 39 |
| 4 | 9 | PG-V + Cimetidine | 5 mg/kg + 50 mg/kg (x3) | 6.2 ± 2.8 | 34 | 66 |

EXAMPLE IV

| Group | No. | TREATMENT (PG) Compound | Dosage Level | RESULT Damage Grading Score | % of Cntrl. | % of Redc'n. |
|---|---|---|---|---|---|---|
| 1 | 12 | veh. cntrl. | 2.5 ml/kg (x3) | 22.7 ± 6.0 | 100 | 0 |
| 2 | 12 | PG-V | 10 mg/kg (x3) | 13.7 ± 3.2 | 60 | 40 |
| 3 | 12 | Cimetidine | 50 mg/kg (x3) | 20.9 ± 5.1 | 92 | 8 |
| 4 | 11 | PG-V + Cimetidine | 10 mg/kg + 50 mg/kg (x3) | 3.6 ± 1.0 | 16 | 84 |

EXAMPLE V

| Group | No. | TREATMENT (PG) Compound | Dosage Level | RESULT Damage Grading Score | % of Cntrl. | % of Redc'n. |
|---|---|---|---|---|---|---|
| 1 | 10 | veh. cntrl. | 2.5 ml/kg (x3) | 22.7 ± 4.6 | 100 | 0 |
| 2 | 11 | PG-V | 10 mg/kg (x3) | 11.9 ± 2.5 | 52 | 48 |
| 3 | 9 | Cimetidine | 50 mg/kg (x3) | 11.6 ± 3.4 | 51 | 49 |
| 4 | 6 | PG-V + Cimetidine | 10 mg/kg + 50 mg/kg (x3) | 4.6 ± 2.3 | 20 | 80 |

EXAMPLE VI

| Group | No. | TREATMENT (PG) Compound | Dosage Level | RESULT Damage Grading Score | % of Cntrl. | % of Redc'n. |
|---|---|---|---|---|---|---|
| 1 | 11 | veh. cntrl. | 2.5 ml/kg (x3) | 19.8 ± 4.2 | 100 | 0 |
| 2 | 12 | PG-W | 10 mg/kg (x3) | 14.8 ± 2.6 | 75 | 25 |
| 3 | 11 | Cimetidine | 50 mg/kg (x3) | 14.1 ± 3.9 | 71 | 29 |
| 4 | 9 | PG-W + Cimetidine | 10mg/kg + 50mg/kg (x3) | 6.7 ± 2.5 | 34 | 66 |

EXAMPLE VII

| | | TREATMENT | | RESULT | | |
|---|---|---|---|---|---|---|
| Group | No. | (PG) Compound | Dosage Level | Damage Grading Score | % of Cntrl. | % of Redc'n. |
| 1 | 7 | veh. cntrl. | 2.5 ml/kg (x3) | 11.6 ± 2.8 | 100 | 0 |
| 2 | 10 | PG-X | 1 mg/kg (x3) | 13.7 ± 4.9 | 118 | -18 |
| 3 | 9 | Cimetidine | 50 mg/kg (x3) | 11.1 ± 5.3 | 96 | 4 |
| 4 | 12 | PG-X + Cimetidine | 1 mg/kg + 50 mg/kg (x3) | 3.9 ± 0.9 | 25 | 75 |

EXAMPLE VIII

| | | TREATMENT | | RESULT | | |
|---|---|---|---|---|---|---|
| Group | No. | (PG) Compound | Dosage Level | Damage Grading Score | % of Cntrl. | % of Redc'n. |
| 1 | 10 | veh. cntrl. | 2.5 ml/kg (x3) | 23.7 ± 4.2 | 100 | 0 |
| 2 | 12 | PG-Y | 10 mg/kg (x3) | 20.8 ± 3.9 | 88 | 12 |
| 3 | 9 | Cimetidine | 50 mg/kg (x3) | 15.7 ± 3.7 | 66 | 34 |
| 4 | 10 | PG-Y + Cimetidine | 10mg/kg + 50mg/kg (x3) | 3.7 ± 2.0 | 16 | 84 |

PG-V in the above Examples is:

9,15 dioxo-7-oxa-prost-13,14-ynoic acid

PG-W in the above Examples is:

15(S)-hydroxy-7-oxa-9-oxo-prost-13,14-ynoic acid

PG-X in the above Examples is:

9-15-dioxo-prost-13,14-ynoic acid

(also known as 11-deoxy-13,14-didehydro-15-keto $PGE_1$, as used herein)

PG-Y in the above Examples is:

9 $\alpha$-Hydroxy-7-oxa-15-oxo-prost-13,14-ynoic acid

The term "No." in the above examples refers to the number of animals in this group.

As can be seen from the above, the administration of a gastric cytoprotective prostaglandin or prostaglandin-like compound in combination with a histamine-2 receptor blocking anti-secretory agent shows a reduction in the gastric inflammatory condition which is beyond the reduction which would be expected based on the efficacy of each of the two agents used separately. The dosage levels administered above are far below the level which exhibits maximal intrinsic activity for the prostaglandin as a cytoprotective agent or the cimetidine as an anti-secretory agent. In some cases, there was no protection afforded at the level administered. Yet, a significant level of protection resulted from the combination.

Similar results to those observed above are obtained when the prostaglandin or prostaglandin-like compound in the above experiments is replaced, in whole or in part, by a biologically equivalent amount of $PGE_1$, $PGE_2$, 15,15-dimethyl $PGE_2$, $PGA_1$, $PGB_1$, $PGF_{2\alpha}$, $PGF_{2\beta}$, $PGD_1$ or pharmaceutically-acceptable salts and esters of these compounds. Similar results are also observed where the cimetidine used in the above experiment is replaced, in whole or in part, by a pharmaceutically-equivalent amount of ranitidine.

## EXAMPLE IX

Using techniques well known in the art, capsules containing the following components are formulated. These capsules, when taken one to four times per day, provide effective protection from peptic ulceration.

| Ingredient | Milligrams per Capsule |
|---|---|
| 9,15 dioxo-7-oxa-prost-13,14-ynoic acid | 2.0 mg |
| Cimetidine | 150.0 mg |
| Lactose Hydrous USP | 100.0 mg |
| Magnesium Stearate USP | 1.5 mg |

EXAMPLE X

Using techniques well known in the art, capsules having the following components are formulated. These capsules, when taken one to four times per day, provide effective protection from peptic ulceration.

| Ingredient | Milligrams per Capsule |
|---|---|
| 9,15 dioxo-prost-13,14-ynoic acid | 0.5 mg |
| Cimetidine | 100.0 mg |
| Lactose Hydrous USP | 100.0 mg |
| Magnesium Stearate USP | 1.5 mg |

CLAIMS:

1. A composition for reducing and preventing gastric inflammatory disease in a human or lower animal at risk to said disease characterized in that it comprises:

(a) a safe and potentiating non-anti-secretory amount of a gastric cytoprotective prostaglandin or prostaglandin-like compound; and

(b) a safe and potentiable amount of a histamine-2 receptor blocking anti-secretory agent;

further characterized in that said gastric cytoprotective prostaglandin is present at a level below which said prostaglandin unilaterally demonstrates maximal cytoprotective intrinsic activity; and said histamine-2 receptor blocking anti-secretory agent is present at a level below which said anti-secretory agent unilaterally demonstrates maximal intrinsic activity as an anti-secretory agent.

2. A composition according to claim 1 wherein said gastric cytoprotective prostaglandin is present at a level of about 0.25% to about 50% of the lowest level of said prostaglandin that unilaterally demonstrates maximal cytoprotective intrinsic activity.

3. A composition according to claim 1 or 2 wherein said histamine-2 receptor blocking anti-secretory agent is administered at a level of about 1% to about 50% of the lowest level that unilaterally demonstrates maximal intrinsic activity as an anti-secretory agent.

4. A composition according to claim 1, 2, or 3 wherein the prostaglandin is selected from the group consisting of $PGE_1$, $PGE_2$, 15,15-dimethyl $PGE_2$, 15,15-dimethyl $PGE_2$, methyl ester, methyl-(S)-15-hydroxy-7-oxa-9-oxo-prost-13,14-ynoate, 15-hydroxy-7-oxa-9-oxo-prost-13,14-ynoic acid, methyl-15-oxo-7-oxa-9-oxo-prost-13,14-ynoate, 15-oxo-7-oxa-9-oxo-prost-13,14-ynoic acid, the 11-deoxy-13,14-dihydro-15-oxo analogue of $PEG_1$, the 11-deoxy-13,14-dihydro-15-oxo analogue of $PGE_2$, $PGA_1$, $PGB_1$,

$PGF_{2\beta}$, $PGF_{2\alpha}$, and $PGD_1$; as well as pharmaceutically-acceptable salts and esters thereof, and mixtures thereof

5. A composition according to claim 1,2, 3, or 4 wherein the histamine-2 receptor blocking anti-secretory agent is selected from the group consisting of cimetidine, ranitidine, and mixtures thereof.

6. A composition according to claim 1, 2, 3, 4, or 5 wherein the histamine-2 receptor blocking anti-secretory agent is cimetidine.

7. A composition according to 1,2,3,4 or 5 wherein the histamine-2 receptor blocking anti-secretory agent is ranitidine.

8. A composition according to Claim 1, 2, 3, 4, 5, 6, or 7 wherein the gastric cytoprotective prostaglandin is 9,15-dioxo-prost-13,14-ynoic acid.

9. A composition for reducing and preventing gastric inflammatory disease in a human or lower animal at risk to said disease characterized in that it comprises:

(a) a safe and potentiating non-anti-secretory level of the gastric cytoprotective prostaglandin 9,15-dioxo-prost-13,14-ynoic acid at a level of about 0.15% to about 50% of the lowest level of said prostaglandin that unilaterally demonstrates maximal cytoprotective intrinsic activity; and

(b) about 1.5 mg to about 150 mg of cimetidine.

0108452

European Patent Office

# EUROPEAN SEARCH REPORT

Application number

EP 83 20 1551

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | CHEMICAL ABSTRACTS, vol. 97, no. 5, 2nd August 1982, pages 31-32, no. 33252q, Columbus, Ohio, USA A. MATERIA et al.: "Prostaglandin E2 potentiates the protective effect of cimetidine against stress-induced gastric ulceration" & J. SURG. RES. 1982, 32(5), 471-476 * Abstract * | 1-9 | A 61 K 31/557<br>A 61 K 45/06 //<br>(A 61 K 31/557<br>A 61 K 415 ) |
| A,D | US-A-4 097 603 (ANDRE ROBERT) | 1-9 | |
| A,D | US-A-3 928 588 (ANDRE ROBERT) | 1-9 | |
| A,D | US-A-3 927 213 (WILBUR LIPPMANN) | 1-9 | TECHNICAL FIELDS SEARCHED (Int. Cl. 3) |
| A,D | US-A-3 903 297 (ANDRE ROBERT) | 1-9 | A 61 K |
| A,D | US-A-3 781 429 (RUTH PARTRIDGE) | 1-9 | |

The present search report has been drawn up for all claims

| Place of search | Date of completion of the search | Examiner |
|---|---|---|
| THE HAGUE | 09-02-1984 | BRINKMANN C. |

CATEGORY OF CITED DOCUMENTS

- X : particularly relevant if taken alone
- Y : particularly relevant if combined with another document of the same category
- A : technological background
- O : non-written disclosure
- P : intermediate document
- T : theory or principle underlying the invention
- E : earlier patent document, but published on, or after the filing date
- D : document cited in the application
- L : document cited for other reasons
- & : member of the same patent family, corresponding document